Europäisches Patentamt

European Patent Office

Office européen des brevets

(11) Numéro de publication: **0 345 122**
**A1**

## (12) DEMANDE DE BREVET EUROPEEN

(21) Numéro de dépôt: **89401422.4**

(22) Date de dépôt: **24.05.89**

(51) Int. Cl.⁴: **C 12 N 1/20**
**A 01 N 63/00**

(30) Priorité: **24.05.88 FR 8806891**

(43) Date de publication de la demande:
**06.12.89 Bulletin 89/49**

(84) Etats contractants désignés:
**AT BE CH DE ES FR GB GR IT LI LU NL SE**

(71) Demandeur: **INSTITUT NATIONAL DE LA RECHERCHE AGRONOMIQUE**
**145, rue de l'Université**
**F-75341 Paris Cédex 07 (FR)**

(72) Inventeur: **Trigalet, André**
**4, rue Eugène Humbert Guitard**
**F-31500 Toulouse (FR)**

Demery, Danielle
4, rue Eugène Humbert
F-31500 Toulouse (FR)

Boucher, Christian
Chemin du Barou Auzielle
F-31650 Saint Orens de Gameville (FR)

Barberis, Patrick
Quartier Saint Martin
F-31450 Bazlège par Montgiscard (FR)

Arlat, Matthieu
8 Boulevard Bonrepos
F-31000 Toulouse (FR)

(74) Mandataire: **Peaucelle, Chantal et al**
**S.C. Ernest Gutmann - Yves Plasseraud 67, boulevard Haussmann**
**F-75008 Paris (FR)**

(54) **Lutte biologique contre des bactéries phytopathogènes.**

(57) Pour lutter contre les bactéries phytopathogènes, on utilise des mutants avirulents dérivés de souches pathogènes, ces mutants comportant une altération résultant d'un évènement de mutagenèse dans les régions de leur génome impliquées dans le pouvoir pathogène.

EP 0 345 122 A1

EP 0 345 122 A1

**Description**

## LUTTE BIOLOGIQUE CONTRE DES BACTERIES PHYTOPATHOGENES

L'invention concerne un procédé de lutte biologique contre Pseudomonas solanacearum et les différents pathovars de Xanthomonas campestris (1), dans lequel on utilise des mutants avirulents dérivés de ces bactéries par altération des gènes de pathogénécité (les références bibliographiques sont données en fin de description). P.solanacearum et X.campestris sont responsables des dégâts les plus importants causés aux cultures à travers le monde. Ainsi, au moins trente familles botaniques, avec plus de deux cents espèces, sont naturellement sensibles à la bactériose produite par P.solanacearum.

Des cultures d'importance économique considérable atteintes par P.solanacearum, comprennent les Solanées, par exemple le tabac, la pomme de terre, la tomate, le poivron, l'aubergine, ou encore les Musacées, comme le bananier. Les souches de X.campestris s'attaquent notamment aux citrus, au coton et au riz.

Selon le processus d'infection par Pseudomonas solanacearum, la souche sauvage virulente pénètre dans la plante-hôte, au niveau des ébauches des racines secondaires. Les bactéries gagnent ensuite le système vasculaire (vaisseaux du bois). Leur propagation rapide à l'intérieur de la plante provoque un flétrissement irréversible.

Divers moyens ont été proposés pour tenter de réduire les graves conséquences socio-économiqomiques de ces bactérioses.ourants dans le cas de Pseudomonas solanacearum consiste à utiliser des variétés résistantes. Cependant, en raison de la nature polygénique de la résistance vis-à-vis de cette bactériose ainsi que de la grande variabilité du pouvoir pathogène exprimée dans les diverses régions du monde, l'obtention de variétés résistantes met en jeu des méthodes longues, de mise en oeuvre difficile et aléatoires.

D'autres méthodes sont basées sur les procédés de lutte biologique. Le principe de ces procédés consiste à installer, au niveau racinaire, et particulièrement à l'intérieur des vaisseaux du bois, un mutant avirulent dérivé de la souche virulente dont la présence doit empêcher l'invasion ultérieure d'une souche virulente soit par antagonisme direct, notamment, par compétition ou production de bactériocines, soit en stimulant un état général de défense chez la plante-hôte.

Dans le cas de P.solanacearum, les essais préliminaires décrits chez la pomme de terre par Kempe et Sequeira dans (2) et chez le tabac par Tanaka (3) s'appuient sur l'utilisation de mutants avirulents spontanés et de Pseudomonas saprophytes du sol.

Cependant, l'installation de ces mutants à l'intérieur des tissus de la plante à protéger reste très limitée, conférant une faible protection.

L'invention est limitée dans le temps car les mutants spontanés ne se multiplient pas dans la plante. Au niveau des racines, en dehors des tissus de la plante, ils sont soumis à un antagonisme microbien qui peut être important dans le sol.

Un pouvoir colonisateur élevé a été en revanche rapporté par Trigalet et Demery (4) chez la tomate avec des mutants avirulents dérivés de la souche virulente GMI1229 de P.solanacearum induits par insertion ponctuelle du transposon Tn5 selon (5). Il s'agit d'une insertion ponctuelle de l'élément transposable dans l'un des gènes impliqués dans le pouvoir pathogène de la bactérie, la plupart de ces gènes étant regroupés et localisés sur un mégaplasmide. Un cosmide appelé pVir2, portant une grande partie de cette région a pu être isolé (7).

Les travaux effectués ont montré que certains de ces mutants sont capables de pénétrer par le système racinaire et de se multiplier à des degrés divers dans la tomate.

La poursuite de ces recherches par les inventeurs a montré que des mutants de ce type peuvent constituer d'excellents compétiteurs de la souche virulente in planta. Le développement de ces travaux a permis d'élaborer, par altération des gènes de pathogénicité, des mutants utilisables dans la lutte biologique contre Pseudomonas solanacearum et les différents pathovars de Xanthomonas campestris.

L'invention a donc pour but de fournir de nouveaux mutants non pathogènes dotés d'un fort pouvoir compétiteur vis-à-vis de la souche virulente in planta.

Elle a également pour but de décrire l'obtention de mutants parfaitement identifiés génétiquement et d'obtention aisée par recombinaison génétique.

L'invention vise également à fournir un procédé de lutte biologique de grande efficacité contre les bactéries phytopathogènes.

Les mutants selon l'invention proviennent de bactéries phytopathogènes dont les gènes impliqués dans le pouvoir pathogène renferment des séquences de nucléotides capables de s'hybrider avec des gènes hrp (gènes de pathogénicité et de réponse hypersensible) de P.solanacearum qui sont principalement regroupés dans une région du génome de P.solanacearum.

L'expression "capables de s'hybrider" telle qu'utilisée dans la description et les revendications signifie que les séquences de nucléotides visées s'hybrident avec des séquences homologues dans les conditions d'hybridation décrites par exemple dans "Current Protocols in Molecular Biology", par Ausubel et al, 1987, John Wiley and Sons, Section IV, unit 2.9, pages 2.9.1 à 2.9.10 et Section II, Unit 6.3, pages 6.3.1 à 6.3.6.

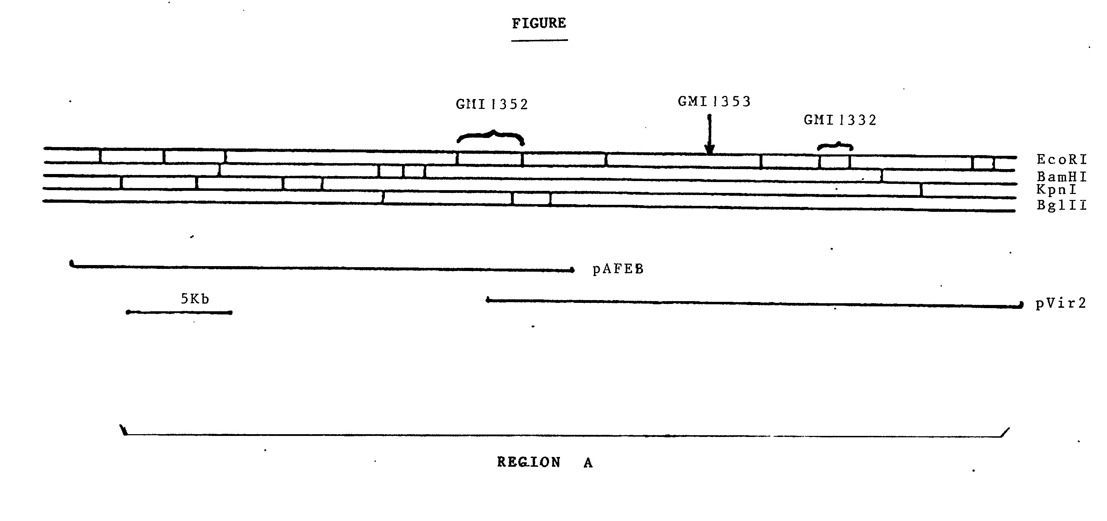

Ces mutants sont caractérisés en ce qu'ils sont non pathogènes, capables de pénétrer dans la plante-hôte et de s'y multiplier, dotés de propriétés compétitrices vis-à-vis de bactéries phytopathogènes dont ils sont dérivés, et qu'ils comportent soit une insertion constituée par une séquence de nucléotides écologiquement acceptable, soit une délétion, ou encore tout autre altération résultant d'un évènement de mutagénèse dans les régions de leur génome impliquées dans le pouvoir pathogène, ces régions comportant des séquences de

2

nucléotides capables de s'hybrider avec la région a) représentée sur la figure 1 regroupant la majorité des gènes hrp actuellement connus, comprenant une séquence d'environ 30 kb du génome de la souche GMI1000 de P.solanacearum qui correspond à l'insert du cosmide pVir2 à laquelle s'ajoute une séquence adjacente de 5 kb du cosmide pAFE8.

De tels mutants constituent des compétiteurs de grand intérêt des souches virulentes à l'intérieur de la plante-hôte.

Le pouvoir compétiteur qu'ils exercent vis-à-vis des souches virulentes est plus stable et plus durable que celui d'un mutant avirulent spontané.

D'une manière avantageuse, les mutations ponctuelles (insertion), les délétions et autres altérations résultant d'un évènement de mutagénèse, dans la région a) définie ci-dessus, ou une région homologue d'une séquence de nucléotides capable de s'hybrider avec la région a) sont contrôlées et reproductibles. Elles permettent ainsi l'élaboration de mutants antagonistes de souches virulentes indigènes, parfaitement adaptés à une zone de culture donnée.

Le région a) évoquée plus haut est caractérisée en ce qu'elle comporte des sites de restriction dans l'ordre suivant :

EcoRI- KpnI- EcoRI-KpnI- BamH1- EcoRI -KpnI- KpnI- BamHI- BgIII-BamHI- BamHI- EcoRI- BgIII-EcoRI- BgIII-EcoRI- EcoRI-EcoRI- EcoRI - EcoRI-BamH1- KpnI- EcoRI- EcoRI.

Des mutants protecteurs de grande efficacité comportent une altération dans la séquence correspondant au quatrième fragment EcoRI de la région a).

D'autres mutants protecteurs préférés comportent une insertion ponctuelle ou une délétion dans le sixième fragment EcoRI.

D'autres mutants encore présentent une altération dans le huitième fragment de cette région.

Dans un grand nombre de souches virulentes de P.solanacearum et des différents pathovars de X.campestrris, la région a) est avantageusement conservée avec une bonne homologie. Quelles que soient les différences intervenues dans l'évolution des bactéries, les séquences correspondant à cette région de pathogénicité sont toutefois capables de s'hybrider avec les gènes hrp de P.solanacearum GMI1000. Tout mutant de P.solanacearum et de X.campestris affecté dans la région homologue à la région précédemment définie, quelque soit la souche virulente dont il dérive, entre dans le cadre de l'invention.

Des mutants dotés d'un pouvoir protecteur élevé vis-à-vis des souches sauvages dérivent de P.solanacearum GMI1229 ou P.solanacearum GMI1000. Il s'agit plus spécialement d'un gène non transposable.

Les insertions ponctuelles réalisées conformément à l'invention correspondent en particulier à des fragments d'ADN portant au moins un gène marqueur. Il s'agit plus spécialement d'un gène non transposable.

De nombreux marqueurs, dès lors qu'ils sont écologiquement acceptables et qu'en particulier ils ne confèrent pas de résistance aux antibiotiques portée sur un élément transposable, peuvent être utilisés. Le choix sera naturellement effectué en fonction des propriétés que l'on souhaite conférer aux mutants protecteurs.

Avantageusement, on introduit des insertions de cassettes conférant aux mutants l'aptitude à métaboliser un substrat carboné, une résistance à un sel de métal lourd, une résistance non transposable à un antibiotique, ou encore des propriétés de bioluminescence.

Par cassette, on entend en effet dans la suite de la description, tout fragment d'ADN portant un gène codant pour la résistance à un antibiotique, ou un gène codant pour la résistance à un métal lourd, ou un gène conférant l'aptitude à métaboliser un substrat carboné.

Des séquences appelées communément "cassettes lac" peuvent être ainsi introduites dans le site désiré de la région a. Ces insertions sont effectuées en opérant selon les techniques classiques du génie génétique.

D'autres cassettes utilisables dans le cadre de l'invention sont par exemple du type de celles figurant dans le catalogue de Pharmacia-LKB Biotechnology, 1989. Il s'agit notamment des cassettes K XIX, KISS, KSAC, KAPA, Kanamycine Resistance Gene Block (ECORI), tetracycline gene block (EcoRI, Aval), chloramphenicol, acetyltransferase gene block, gène gus (glucuronidase) d'E.coli K12, gène lac Z d'E.coli, gène PNEO dominant selectable marker vector.

Des cassettes ou familles d'interposons également utilisable correspondent à celles décrites dans (8), tels que les fragments Ω : (Ω - Sm/Spc, Ω -Km, Ω - Tc, Ω - Cm, Ω - Hg ou Ω - Ap.

On notera que la région des gènes hrp évoquée ci-dessus est utilisable comme sonde pour isoler des gènes de séquence homologues, contenus dans une banque de gènes d'une souche bactérienne autre que la souche P.solanacearum GMI1000. On réalise dans ce cas une construction génétique, selon les techniques classiques, sur ces gènes, puis on les réintroduit dans la souche bactérienne à utiliser selon l'invention.

Des mutants protecteurs avirulents comportant une insertion Tn5 dans la région des gènes hrp ont été préparés selon l'invention. A partir de ces mutants, il est possible de construire des mutants protecteurs avirulents écologiquement acceptables par mutagénèse dirigée en opérant par exemple comme décrit dans (7). Il s'agit donc de mutants dans lesquels l'altération se situe dans la région d'insertion de Tn5.

D'une manière générale, les mutants de l'invention sont construits par altération de la région du génome regroupant les gènes hrp, en ayant recours aux techniques classiques, notamment à celles décrites dans (7).

La manipulation génétique dans le locus d'insertion du transposon Tn5 est induite par l'introduction d'une cassette, par une délétion ou par tout autre évènement de mutagénèse.

L'insertion du transposon Tn5 chez le mutant GMI1353 se situe dans un fragment EcoRI d'environ 8 kb, qui correspond au fragment 6 sur la figure, présentant la carte de restriction suivante. Entre parenthèses, on indique la distance exprimée en kb, séparant 2 sites :

*Eco*RI *(0,2)- Sst*I *(0,9)- Sma*I *(0,2)- Cla*I *(0,9)- Not*I *(0,2)- Sst*I *(1,5)- Sma*I *(1,3)- Sma*I, *Eco*RV, *(2,9)- Eco*RI.

L'insertion du transposon Tn5 se situe dans le dernier fragment *Sma*I, *Eco*RV-*Eco*RI de 2,9 kb. L'insertion du transposon Tn5 dans ce fragment est très proche d'un site EcoRV.

Des mutants ont été obtenus dans ce fragments:

1. Après digestion par *Eco*RV et insertion d'une cassette,
2. Après délétion du fragment *Sma*I-*Sma*I de 1,3 kb et religature ou remplacement par une cassette,
3. Par délétion du fragment *Sma*I-*Eco*RI de 2,9 kb et religature, ou remplacement par une cassette,
4. Par délétion du fragment *Sma*I-*Sma*I-*Eco*RI de 4,2 kb et religature, ou insertion d'une cassette.

Dans ces deux derniers exemples, la religature a été effectuée après transformation en extrémité bout franc par remplissage enzymatique approprié de l'extrémité cohésive *Eco*RI.

En variante, les mutants de l'invention comportent, comme indiqué ci-dessus, une délétion dans la région de leur génome impliquée dans le pouvoir pathogène de la souche virulente. Ces délétions sont réalisées, de manière habituelle, in vitro, par coupure à des sites de restriction, suivie de religature. On utilise par exemple des exonucléases telles que Bal31 ou l'exonucléase III. D'autres exonucléases utilisables correspondent notamment à celles figurant dans les catalogues de Pharmacia, BRL, Boehringer Manheim, Amersham).

Les mutants ainsi obtenus sont avirulents chez la tomate et induisent une protection de 70 à 90% contre l'inoculation différée de 8 jours par la souche virulente GMI1229 dans les conditions définies dans le tableau 7.

Ces mutants induisent notamment une protection contre l'inoculation différée de 8 jours par la souche virulente GMI1229 (de50 à 70% de protection pour les mutants dérivés de GMI1332, de 60 à 85% pour les dérivés de GMI1252) dans les conditions définies dans le tableau 7.

L'insertion du transposon Tn5 chez le mutant GMI1332 se situe dans un fragment d'environ 1,5 kb et celle du mutant GMI1352 dans un fragment *Eco*RI d'environ 2,0 kb (voir figure).

Des mutants ont été obtenus à partir du fragment cloné des mutants ou des fragments homogues clonés à partir de la souche virulente de ces deux mutants GMI1332 et GMI1352 par délétion, après digestion *Eco*RI et religature, ainsi que par insertion d'une cassette.

Une autre modification génétique a été entreprise chez chacun de ces 3 mutants GMI1332, GMI1352 et GMI1353, en tenant compte du fait que le transposon Tn5 possède 2 sites *Hpa*I dans les séquences d'insertion.

Le fragment cloné, contenant le locus porteur du Tn5 a été digéré par *Hpa*I, puis religaturé sur lui-même. Dans une autre série d'expérience, une cassette ($Km_R$) a été insérée à la place du transposon Tn5.

Ces constructions stables peuvent être avantageusement introduites par recombinaison homologue dans une souche quelconque de P.solanacearum ou des différents pathovars de X.campestris pourvu qu'il existe un homologie suffisante entre les séquences d'ADN.

L'étude in vitro des propriétés protectrices des mutants définis ci-dessus vis-à-vis de souches virulentes pathogènes a permis de mettre en évidence leur grande efficacité.

Les essais dont les résultats sont rapportés ci-après montrent que dans des conditions expérimentales de développement maximum de la maladie, les mutants de l'invention assurent une protection pouvant atteindre 100%. Ces résultats mettent en valeur le caractère hautement compétitif des mutants de l'invention par rapport aux mutants avirulents de l'art antérieur.

L'invention vise donc également des compositions d'inoculum pour la lutte biologique contre les bactéries phytopathogènes caractérisées en ce qu'elles renferment de tels mutants.

Ces compositions sont constituées avantageusement de suspensions des cellules de mutants dans un milieu approprié, plus spécialement dans de l'eau. En variante, elles se présentent sous forme de granulés comprenant les cellules des mutants enrobées par un véhicule solide.

Pour la préparation de ces granulés, on aura avantageusement recours à la technique décrite dans la demande FR 2615203 concernant le conditionnement d'inoculum de micro-organismes sous forme de granulés biodégradables.

De nombreuses autres méthodes de préparations de compositions d'inoculums bactériens sont décrites.

La concentration de ces compositions est avantageusement telle qu'une fois appliquées, le rapport entre le mutant et la population naturelle virulente est d'au moins environ 100.

Cette concentration sera aisément ajustée par l'homme de l'art en tenant compte, notamment, des véhicules liquides ou solides utilisés et de la partie de la plante traitée (graines tubercules, racines, feuilles).

Les mutants utilisés pour élaborer ces compositions proviennent de cultures effectuées dans les mêmes conditions que les souches virulentes, sur milieu physiologique glucosé classique.

L'invention vise en outre un procédé de lutte biologique contre les bactéries phytopathogènes, plus spécialement contre P.solanacearum et les différents pathovars de X.campestris.ection des plantes contre les maladies dues à P.solanacearum et aux différents pathovars de X.campestris.

Ce procédé de protection est caractérisé en ce qu'on inocule un mutant tel que défini ci-dessus, ou une composition le renfermant.

Pour l'application, on inocule avantageusement une suspension de mutant, en particulier une suspension aqueuse, en arrosant à la racine les plantes à traiter.

Cette application est réalisée de préférence à un stade précoce du développement pour établir une invasion rapide de la plante, plus spécialement au stade pépinière, sur les semences (graines, tubercules), plantules, plants, boutures.

En variante, on utilise comme indiqué ci-dessus des granulés ou toute autre forme appropriée connue de l'homme de l'art.

Le procédé de l'invention permet de lutter de manière efficace contre les dégats causés aux cultures, en particulier aux cultures de Solanées et de Musacées par P.solanacearum et des différentes plantes hôtes des différents pathovars de X.campestris..

La vaste répartition géographique de ces bactérioses dans le monde et la gamme d'hôte étendue concernée permettent de mesurer l'intérêt des nouveaux moyens de protection fournis par l'invention.

D'autres caractéristiques et avantages de l'invention apparaîtront dans la description qui suit d'essais réalisés sur la tomate et en se reportant à la figure 1 qui représente la carte de restriction de la région a mention des sites d'insertion chez les mutants.

Les mutants des essais rapportés ci-après répondent à l'ensemble des caractéristiques des mutants de l'invention mais comportent une insertion Tn5 portant un gène de résistance à un antibiotique. Ils illustrent cependant l'invention dès lors qu'ils correspondent aux mutants de l'invention sur un plan structurel.

## I.MATERIELS ET METHODES

### Souches bactériennes :

Comme souche pathogène de référence, on a utilisé P.solanacearum GMl1229. Cette souche est résistante à la streptomycine ($Sm_r$) et à la rifampicine ($Rif_r$).

Les mutants Tn5 avirulents dérivent de cette souche et sont de plus résistants à la kanamycine ($Km_r$).

### Inocolum bactérien :

Les bactéries sont cultivées sur un milieu TTC gelosé puis en phase liquide dans un milieu physiologique comme décrit dans (5). L'inoculum est préparé à partir d'une préculture en phase iliquide, dans des Erlens d'un litre contenant 400 ml de milieu, sur un agitateur Kotterman indice n° 6, à 30°C. Les bactéries sont récoltées à la fin de leur phase exponentielle de croissance soit 30 heures après l'ensemencement du milieu. L'inoculum est ensuite ajusté à la concentration désirée en fonction d'une mesure de la densité optique à 620 nm (colorimètre Jouan AE.11) contrôlée par une numération bactérienne à l'aide d'un ensemenceur spirale (Spiral plate Maker model D, Interscience, France).

### Matériel végétal :

On a utilisé alternativement des cultivars sensibles de tomate H63-5 et Supermarmande.

### Inoculum :

Les plants de tomate ont été inoculés après avoir atteint environ 15 cm en hauteur (stade 4 à 5 feuilles). Les plants de tomate contenus dans des pots en tourbe ont été mis en place, à raison de 12 plants, dans des bacs (30 x 40 x 10 cm) contenant le mélange tourbe/terreau horticole décrit dans (4). Pour l'inoculation, on a arrosé au pied de chaque plant avec 100 ml de suspension bactérienne ajustée à la concentration désirée. Les bacs ont été ensuite conservés dans une chambre d'incubation dans les conditions suivantes : 18 heures d'éclairement 120 microEinstein/s/m$_2$, humidité relative 90%, température + 30°C ; suivies d'une période de nuit de 6 heures, température + 28°C, humidité relative 90 à 100%.

### Notation des résultats :

L'évaluation du développement de la maladie a été effectuée selon l'échelle de 0 à 5 préconisée par Winstead et Kelman dans (6) et utilisée dans les essais de lutte biologique décrits par Kempe et Sequeira dans (2) et par Tanaka dans (3). Cette échelle est la suivante : indices de maladie :

0, aucun symptôme externe de maladie,

1, jusqu'à 25% du feuillage est flétri,

2, de 25% à 50%

3, de 50% à 75%

4, de 75% à 100%

5, l'ensemble du plant est totalement flétri.

L'indice moyen de maladie (IM) est alors défini par la relation suivante :

$IM = (0.no + 1.n_1 + 2.n_2 + 3.n_3 + 4.n_4 + 5.,_5)/N$ où N est le nombre total de plants inoculés, et $n_i$ (i variante de 0 à 5) est le nombre de plants montrant l'indice de maladie correspondant de 0 à 5 respectivement.

Le pourcentage de protection est défini selon Tanaka (3) :

$$\% \ P = \left[ 1 - \frac{IMp}{IM^*} \right] 100$$

$IMp$ = indice moyen de la maladie dans l'expérience (mutant + sauvage),

$IM^*$ = indice moyen de la maladie chez le témoin sauvage.

Isolements

Des isolements ont été effectués sur des plants sans symptômes externes apparents, à différents niveaux dans la plante, et à différents temps après l'inoculation, comme décrit dans (4).

II. EXPERIENCES SUR DES PLANTS DE TOMATE SENSIBLES.cv.H63-5 (STADE 3 à 5 FEUILLES, 18 PLANTS/TRAITEMENT).

a-

Ces plants ont été arrosés par un mélange de suspensions de P. Solanacearum contenant des quantités comparables 2 x $10_9$ CFU : $mL_{-1}$ (bactéries viables/ml) d'un mutant Tn5 avirulent et de la souche virulente GMI1229.

Les indices moyens de maladie obtenus à différents temps après l'inoculation de ces mélanges sont rapportés dans le tableau 1 ci-après :

TABLEAU 1

| | $t_0$ | +7 | +12 | +2C | +30 | +50 jours |
|---|---|---|---|---|---|---|
| Eau distillée | | 0,0 | 0,0 | 0,C | 0,0 | 0,0 |
| Sauvage GMI1229 | | 1,1 | 5,0 | 5,C | 5,0 | 5,0 |
| Mélange souche virulente/mutant | | | | | | |
| /GMI1360 | | 1,1 | 1,6 | 2,2 | 5,0 | 5,0 |
| /GMI1358 | | 0,5 | 2,2 | 2,5 | 5,0 | 5,0 |
| /GMI1332 | | 0,7 | 2,2 | 2,7 | 5,0 | 5,0 |
| /GMI1333 | | 0,0 | 1,1 | 2,7 | 5,0 | 5,0 |
| /GMI1355 | | 0,0 | 0,5 | 1,· | 5,0 | 5,0 |
| /GMI1357 | | 0,0 | 2,2 | 3,2 | 4,4 | 5,0 |
| /GMI1353 | | 0,5 | 1,1 | 2,2 | 3,0 | 3,8 |
| /GMI1351 | | 0,5 | 1,1 | 2,7 | 2,7 | 3,3 |
| /GMI1350 | | 0,0 | 0,3 | 0,5 | 0,8 | 1,4 |
| /GMI1352 | | 0,5 | 3,3 | 5,C | 5,0 | 5,0 |

Tomates cv.H63-5, · stade à 5 feuilles, 18 plants/traitement.

L'examen de ces résultats montre que dans ces conditions, l'inoculation simultanée d'un mélange mutant Tn5 avirulent/souche virulente se traduit par un retard dans l'évolution de la maladie, comparée à celle observée avec le témoin GMI1229. En effet, on observe avec ce témoin 100% de flétrissure (IM = 5) en 12 jours seulement alors que la totalité des lots inoculés par le mélange mutant Tn5/souche virulente n'exprime 100% de maladie qu'au bout de 30 jours. Trois mutants semblent montrer un pouvoir protecteur supérieur aux autres, à savoir GMI1353, GMI1351 et GMI1350, dans la mesure où 50 jours après l'inoculation mixte l'indice moyen de maladie varie de 3,8 à 1,4.

b-

Dans une autre série d'expériences, on a vérifié l'influence de la concentration relative du mutant Tn5 avirulent par rapport à celle de la souche virulente, sur l'indice moyen de la maladie.

Les plants de tomate cv.H63-5 (stade 3 à 5 feuilles, 18 plants/traitement) ont été inoculés avec des mélanges de suspensions de P. solanacearum, les concentrations en mutant Tn5/souche virulente étant dans des rapports quantitatifs de 1 et de 10.

On rapporte dans le tableau 2 ci-après les résultats obtenus avec les trois mutants GMI1353, GMI1351 et GMI1350.

6

TABLEAU 2

| | $t_0$ | +7 | +20 | +35 | jours |
|---|---|---|---|---|---|

| | +7 | +20 | +35 |
|---|---|---|---|
| Eau distillée | 0,0 | 0,0 | 0,0 |
| Sauvage GMI1229[c1] | 2,2 | 5,0 | 5,0 |
| " GMI1229[c2] | 1,6 | 5,0 | 5,0 |
| Mélange virulent/mutant | | | |
| 1229[c1]/1353[c1] | 2,2 | 3,3 | 3,3 |
| 1229[c1]/1351[c1] | 0,5 | 1,1 | 2,7 |
| 1229[c1]/1350[c1] | 0,0 | 1,4 | 1,9 |
| 1229[c2]/1353[c1] | 1,6 | 2,2 | 2,2 |
| 1229[c2]/1351[c1] | 0,0 | 1,1 | 1,7 |
| 1229[c2]/1350[c1] | 0,0 | 0,5 | 1,0 |

$c1 : 2 \times 10^9 \text{ CFU-ml}^{-1}$

$c2 : 2 \times 10^8 \text{ CFU-ml}^{-1}$

On constate que si le mélange contient dix fois plus de cellules du mutant avirulent que de cellules de la souche virulente, l'indice moyen de maladie baisse notablement par rapport à l'indice moyen de maladie obtenu à l'aide d'un mélange en quantité comparable.

c-

Dans d'autres expériences réalisées sur des tomates cv.Supermarmande, stade 5 à 8 feuilles (28 plants/traitement), le mutant Tn5 avirulent et la souche virulente ont été inoculés séparément.

Les résultats obtenus figurent dans le tableau 3 ci-après qui donne les indices moyens de maladie obtenus à différents temps après l'inoculation de la souche virulente, différée d'un jour par rapport à celle du mutant, avec différents rapports en concentration mutant/souche virulente. Les concentrations $c_1$ et $c_2$ en cellules de mutant et/ou en cellules de la souche virulente sont respectivement de $2 \times 10_9$ CFU.ml$_{-1}$ et $2 \times 10_8$ CFU.ml$_{-1}$.

7

## TABLEAU 3

|  | +7 | +12 | +25 | +35 |
|---|---|---|---|---|
| $T_O$ jours | | | | |
| Eau distillée | 0,0 | 0,0 | 0,0 | 0,0 |
| Sauvage GMI1229[c1] | 5,0 | 5,0 | 5,0 | 5,0 |
| " GMI1229[c2] | 2,2 | 5,0 | 5,0 | 5,0 |
| Mutant + (différé 1 jour) souche virulente | | | | |
| A 1353[c1]/1229[c1] | 0,7 | 2,2 | 3,3 | 3,6 |
| B 1353[c1]/1229[c2] | 0,0 | 0,0 | 0,7 | 0,7 |
| Isolement | | | | |

Expérience B   IM sauvage = 5

IM$_p$ mutant = 0

% P = 100%

On observe qu'une quantité dix fois plus grande de mutant par rapport à la souche virulente se traduit par une baisse notable de l'indice moyen de maladie. Dans cette expérience, une quantité comparable mutant/souche virulente se traduit au bout de 50 jours par un indice moyen de maladie de 4,6 alors que la protection devient totale (IM = 0) lorsque l'inoculum de mutant Tn5 est dix fois plus concentré que celui de la souche virulente.

Parmi les 28 plants sans symptômes externes de maladie, inoculés par le mutant GMI1353 à la concentration de $2.10_9$ CFU.ml$_{-1}$, puis 24 heures plus tard à l'aide d'une suspension dix fois moins concentrée de la souche sauvage, une série de 10 plants, choisis au hasard, et répertoriés, a donné lieu à des isolements échelonnés dans le temps. Un tronçon de tige de 5 mm d'arête a été prélevé au niveau de la feuille n°3 (+ 20 cm par rapport au niveau du sol) au temps $t_0 + 12$ jours. Un nouveau tronçon a été prélevé au niveau + 18 cm au temps $t_0 + 25$ jours et enfin au niveau + 16 cl au temps $t_0 + 35$ jours. Lors de ce dernier prélèvement tous les plants ont été sacrifiés et un isolement au niveau cotylédonnaire (+5 cm) a été opéré. On a constaté qu'aucun isolement dans la partie aérienne supérieure (feuille n°3) des plants de tomate ne révèle la présence de la souche virulente même 34 jours après l'inoculation de cette dernière.

d -

Dans le tableau 4 qui suit, on rapporte les résultats obtenus concernant les isolements et la numération bactérienne ($log_{10}$) à partir de plants sans symptômes externes apparents après inoculation séquentielle (1 jour) du mutant avirulent GMI1353 puis de la souche virulente GMI1229. Le rapport entre l'inoculum virulent et l'inoculum avirulent est dc 0,1.

Les expériences ont été réalisées sur des tomates cv Super Marmande (120 plants), avec une concentration en inoculum : GMI1353[c1] de $2 \times 10^9$ CFU/ml$^{-1}$ et GMI1229[c2] de $2 \times 10^8$ CFU $\times$ ml$^{-1}$. L'indice de maladie dans la combinaison GMI1353 contre GMI1229 était de 1,75 et l'indice de maladie dans le témoin virulent de 5,0 au douzième jour.

TABLEAU 4

NOMBRE DE JOURS A INOCULATION COMPETITIVE

| | 20 | | 30 | | 45 | |
|---|---|---|---|---|---|---|
| | | | | | temps | |
| Nb de plants analyse | 10 | | 10 | | 58 | |
| | Souche | Mutant | Souche | Mutant | Souche | Mutant |
| Isolement négatif | 9 | 2 | 8 | 2 | 53 | 15 |
| Isolement positif | 1 | 8 | 2 | 8 | 5 | 43 |
| Numération bactérienne dans les plants à isolement positif | | | | | | |
| 2,0 à 3,0 | - | 1 | - | 2 | - | 9 |
| 3,0 à 4,0 | - | 3 | - | 1 | 1 | 11 |
| 4,0 à 5,0 | - | - | - | 2 | - | 5 |
| 5,0 à 6,0 | - | - | - | 3 | - | 6 |
| 6,0 à 7,0 | 1 | 1 | 1 | - | - | 2 |
| > 7,0 | - | 3 | 1 | - | 4 | 10 |

Les résultats indiquent que les isolements, réalisés à partir de plants sans symptômes, traduisent une présence faible de la souche virulente (8 isolements), une présence importante du mutant avirulent (59 isolements) dans l'ensemble des 70 plants analysés au cours du temps.

e -

Plusieurs expériences ont été effectuées sur des plants de tomates cv Super Marmande (36 plants dans chaque expérience) pour étudier les indices de maladie et le pourcentage de protection en inoculant séquentiellement (écart de 8 jours) des mutants Tn5 puis la souche virulente GMI1229, selon différents rapports relatifs d'inoculum.

TABLEAU 5

| INOCULUM | INDICES DE MALADIE NB DE JOURS APRES L'INOCULATION COMPETITIVE | | | % DE PROTECTION |
|---|---|---|---|---|
| | 12 | 30 | 45 | |
| | | | | temps |
| Eau distillée stérile | 0,0 | 0,0 | 0,0 | |
| GMI1229 (b) | 5,0 | 5,0 | 5,0 | |
| GMI1229 (c) | 4,7 | 5,0 | 5,0 | % DE PROTECTION |
| Mutants avirulents (c) GMI1332, 1351, 1352, 1353 | 0,0 | 0,0 | 0,0 | |
| MRI (a) vs GMI1229 (c) | 3,5 | 4,2 | 4,8 | 4 |
| GMI1332 (a) vs GMI1229 | 0,0 | 0,9 | 2,4 | 52 |
| GMI1351 (a) vs GMI1229 | 0,0 | 0,8 | 1,7 | 66 |
| GMI1352 (a) vs GMI1229 (c) | 0,1 | 0,9 | 1,4 | 72 |
| GMI1353 (a) vs GMI1229 (b) | 0,3 | 1,3 | 1,3 | 74 |
| GMI1353 (a) vs GMI1229 (c) | 0,1 | 0,5 | 1,0 | 80 |

Les résultats indiquent que, dans les conditions de l'inoculation, les mutants Tn5 avirulents GMI1332, GMI1351, GMI1352 et GMI1353, confèrent un indice de protection au bout de 45 jours allant de 52% à 80% respectivement, alors qu'un mutant avirulent spontané MRI ne réussit pas à s'installer à l'intérieur de la plante et ne confère que 4% de protection dans les mêmes conditions.

f -

Dans le tableau 6 ci-dessous, on indique les résultats obtenus concernant l'isolement et la numération bactérienne observés sur des plants sans symptômes externes, 45 jours après inoculation compétitive de mutants avirulents Tn5 contre des souches virulentes GMl129 (rapport mutant/souche virulente de 0,01), (inoculation séquentielle avec écart de 8 jours).

Ces expériences ont été réalisées sur des plants de tomates cv Super Marmande (36 plants pour chaque expérience), la concentration en inoculum étant de $2 \times 10^9$ CFU $\times$ ml$^{-1}$ (a) et de $2 \times 10^7$ CFU $\times$ ml$^{-1}$ (c). La numération bactérienne a été effectuée au niveau des cotylédons (positif, en log$_{10}$; - : négative).

TABLEAU 6

| INOCULUM | GMI1332(a) | | GMI1351(a) | | GMI1352(a) | | GMI1353(a) | |
|---|---|---|---|---|---|---|---|---|
| | GMI1229(c) | | GMI1229(c) | | GMI1229(c) | | GMI1229(c) | |
| | avirulent | virulent | avirulent | virulent | avirulent | virulent | avirulent | virulent |
| NB DE PLANTES SANS SYMPTOMES EXTERNES AU 45e JOUR | | 19 | | 24 | | 26 | 25 | 25 |
| ISOLEMENTS NEGATIFS | 11 | 19 | 4 | 23 | 8 | 25 | 7 | 25 |
| ISOLEMENTS POSITIFS | 8 | - | 20 | 1 | 18 | 1 | 18 | - |
| NUMERATION BACTERIENNE $(log_{10})$ | | | | | | | | |
| 2,0 à 3,0 | 1 | - | 4 | - | 2 | - | 1 | - |
| 3,0 à 4,0 | 2 | - | 7 | - | 4 | - | 5 | - |
| 4,0 à 5,0 | 2 | - | 5 | - | 3 | - | 4 | - |
| 5,0 à 6,0 | - | - | 4 | - | 5 | - | 3 | - |
| 6,0 à 7,0 | 3 | - | - | 1 | - | - | 1 | - |
| > 7,0 | - | - | - | - | 4 | 1 | 4 | - |

11

g -

Les indices de maladies et les pourcentages de protection dans le cas d'inoculation séquentielle (8 jours d'écart) du mutant avirulent GMI1353 et de souches virulentes sont rapportés dans le tableau 7. Le rapport des concentrations mutant avirulent/souche virulente est de 0,01.

Ces expériences ont été réalisées sur des plants de tomates cv Super Marmande (36 plants pour chaque expérience), avec des concentrations en inoculum de : (a) : $2 \times 10^9$ CFU.ml$^{-1}$; (c) : $2 \times 10^7$ CFU.ml$^1$.

### TABLEAU 7

| Inoculum | INDICES DE PROTECTION | | | % DE PROTECTION |
|---|---|---|---|---|
| | NB DE JOURS APRES L'INOCULATION COMPETITIVE | | | |
| | 12 | 30 | 45 | |
| Eau distillée stérile | 0.0 | 0.0 | 0.0 | |
| GMI1353 (a) | 0.0 | 0.0 | 0.0 | |
| GMI1229 (c) | 4.7 | 5.0 | 5.0 | |
| K60 (c) | 3.5 | 5.0 | 5.0 | |
| MT1 (c) | 4.8 | 5.0 | 5.0 | |
| GMI1353 (a) vs GMI1229 (c) | 0.2 | 0.7 | 1.4 | 72 |
| GMI1353 (a) vs K60 | 0.1 | 0.5 | 1.1 | 73 |
| GMI1353 (a) vs MT1 | 0.1 | 0.8 | 1.5 | 70 |

Ces résultats montrent que le mutant avirulent GMI1353 présente un effet protecteur vi-à-vis de souches virulentes autres que la souche GMI1229 dont il provient.

Les résultats ci-dessus montrent l'intérêt de l'utilisation de mutants selon l'invention dans la lutte biologique contre les bactéries phytopathogènes, en particulier lorsque le mutant avirulent est inoculé en premier lieu, avec un écart d'au moins un jour avec l'inoculation de la souche virulente.

On notera que les effets protecteurs importants observés dans ces expériences sont obtenus dans des conditions de développement très favorables des maladies (indice de maladie de 5,0 avec le témoin virulent dans les 12 jours suivants l'inoculation).

Ces résultats sont donc améliorés lors de l'utilisation en plein champ où les concentrations en inoculum virulent dans les sols naturels, sont bien inférieures à celles utilisées dans les expériences ci-dessu. De plus, les mutants protecteurs avirulents ayant été établis dans la plante hôte, les conditions de compétition avec la souche virulente jouent en leur faveur.

Les isolements bactériens et les numérations effectués sur des plants sans symptômes apparents montrent que les mutants avirulents colonisent les tissus des plantes et empêchent l'invasion ultérieure et la multiplication d'une souche virulente (tableaux 4 et 6).

### REFERENCES BIBLIOGRAPHIQUES

1. YOUNG et al, N.Z.J. Agri. Res. 1978, 21:153-177
2. KEMPE J. et SEQUEIRA L. ; Plant Disease 1983, 67:499-503
3. TANAKA H. Bull. Utsunomiya Tobacco Exp. Station 1985. 21:1-64
4. TRIGALET A. et DEMERY D. ; Physiol. Mol. Plant. Pathol. 1986, 28:423-430
5. BOUCHER C. et al, J. Gen. Microbiol. 1985, 131:2449-2457
6. WINSTEAD N.N. and KELMAN A. ; Phytopathology 1952, 42:628-634
7. RUVKUN G. et AUSUBEL F., Nature, 1981, 289, 85-88
8. FELLAY R., Frey J., Frisch H., Gene, <u>52</u>, 147-154, 1987

### Revendications

1. Mutants dotés d'un effet protecteur vis-à-vis de bactéries phytopathogènes, telles que <u>P.solanacearum</u> et les différents pathovars de <u>X.campestris</u>, dont les gènes impliqués dans le pouvoir pathogène renferment des séquences de nucléotides capables de s'hybrider avec un gène de pathogénicité de <u>P.Solanacearum</u>, caractérisés en ce qu'ils sont non pathothogènes, capables de pénétrer dans la plante-hôte et et s'y multiplier, dotés de propriétés antagonistes vis-à-vis des bactéries phytopathogènes, et qu'ils comportenortent soit une insertion constituée par une séquence de nucléotides écologiquement acceptables, soit une délétion, ou encore tout autre altération résultant d'un évènement de mutagénèse dans les régions de leur génome impliquées dans le pouvoir pathogène, ces régions comportant des séquences de nucléotides capables de s'hybrider avec la région a) représentée sur la figure 1 regroupant la majorité des gènes hrp comprenant une séquence d'environ 30 kb du génome de la souche GMI 1000 de P.Solanacearum qui correspond à l'insert du cosmide pVir2 à laquelle s'ajoute une séquence adjacente de 5 kb du cosmide pAEF8.

2. Mutants selon la revendication 1, caractérisés en ce que la région a) comporte les sites de restriction dans l'ordre suivant :
EcoRI- Kpnl- EcoRI-Kpnl- BamH1- EcoRI -Kpnl- Kpnl- BamHI- BglII-BamHI- BamHI- EcoRI- BglII-EcoRI- BglII- EcoRI- EcoRI-EcoRI - EcoRI - EcoRI-BamH1- Kpnl- EcoRI- EcoRI

3. Mutants selon la revendication 1 ou 2, caractérisés en ce qu'ils comportent une altération dans la séquence correspondant au quatrième fragment EcoRI, ou au sixième fragment EcoRI ou encore au huitième fragment EcoRI de la région a).

4. Mutants selon l'une quelconque des revendications 1 à 3, caractérisés en ce que les insertions d'ADN portent au moins un gène marqueur, écologiquement acceptable, avantageusement un marqueur capable de conférer aux mutants l'aptitude à métaboliser un substrat carboné, une résistance à un sel de métal lourd ou des propriétés de bioluminescence.

5. Mutants selon la revendication 4, caractérisés en ce qu'ils comprennent une insertion d'ADN au site EcoRV dans un fragment EcoRI d'environ 8 kb, correspondant au fragment 6 sur la figure 1, présentant la carte de restriction suivante, la distance entre deux sites étant exprimée en kb :
EcoRI (0,2)- SstI (0,9)- Smal (0,2)- Clal (0,9)- NotI (0,2)- SstI(1,5)- Smal (1,3)- Smal, EcoRV, (2,9)- EcoRI.

6. Mutants selon l'une quelconque des revendications 1 à 5, caractérisés en ce qu'ils dérivent de P.Solanacearum, notamment des souches GMI1229 ou GMI1000, ou de pathovars de X.Campestris.

7. Compositions pour la lutte biologique contre les bactéries phytopathogènes, caractérisées en ce qu'elles renferment une quantité efficace d'un mutant selon l'une quelconque des revendications 1 à 6, en suspension dans un milieu approprié, plus spécialement de l'eau ou sous forme de granulés.

8. Procédé de lutte biologique contre les bactéries phytopathogènes, caractérisé en ce qu'on inocule dans les plantes un mutant avirulent selon l'une quelconque des revendications 1 à 6 ou une composition selon la revendication 7.

**FIGURE**

GMI 1352  GMI 1353  GMI 1332

EcoRI
BamHI
KpnI
BglII

pAFEB

5Kb

pVir2

REGION   A

EP 0 345 122 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | BIOLOGICAL ABSTRACTS, vol. 82, no. 11, 1986, page 849, résumé no. 106056, Philadelphia, PA, US; A. TRIGALET et al.: "Invasiveness in tomato plants of (transposon) Tn5-induced avirulent mutants of Pseudomonas solanacearum", & PHYSIOL. MOL. PLANT PATHOL. 28(3): 423-430. 1986 * Résumé * | 1-6 | C 12 N 1/20 A 01 N 63/00 |
| Y | IDEM | 7,8 | |
| D,X | CHEMICAL ABSTRACTS, vol. 103, 1985, page 391, résumé no. 192987s, Columbus, Ohio, US; C.A. BOUCHER et al.: "Transposon mutagenesis of Pseudomonas solanacearum: isolation of Tn5-induced avirulent mutants", & J. GEN. MICROBIOL. 1985, 131(9), 2449-57 * Résumé * | 1-6 | |
| Y | IDEM | 7,8 | |
| X | CHEMICAL ABSTRACTS, vol. 106, 1987, page 130, résumé no. 61898c, Columbus, Ohio, US; C. BOUCHER et al.: "Virulence genes are carried by a megaplasmid of the plant pathogen Pseudomonas solanacearum", & MOL. GEN. GENET. 1986, 205(2), 270-5 * Résumé * | 1-6 | |
| Y | IDEM -/- | 7,8 | |

**DOMAINES TECHNIQUES RECHERCHES (Int. Cl.4)**

C 12 N
A 01 N

Le présent rapport a été établi pour toutes les revendications

| Lieu de la recherche | Date d'achèvement de la recherche | Examinateur |
|---|---|---|
| LA HAYE | 04-09-1989 | MADDOX A.D. |

EPO FORM 1503 03.82 (P0402)

Office européen
des brevets

**RAPPORT DE RECHERCHE EUROPEENNE**

Numero de la demande

EP 89 40 1422

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | BIOLOGICAL ABSTRACTS, vol. 78, 1984, colonne de gauche, résumé no. 78579, Phidelphia, PA, US; W.Y. CHEN et al.: "Effects of avirulent bacteriocin-producing strains of Pseudomonas solanacearum on the control of bacterial wilt of tobacco", & PLANT PATHCL. (LOND) 33(2): 245-254. 1984 * Résumé * ---- | 1,7,8 | |
| X | EP-A-0 257 756 (DAIKIN) * Page 12, point (3)-1 - page 13; page 20, exemples 3-1 * | 1 | |
| Y | ---- | 7,8 | |
| D,X | CHEMICAL ABSTRACTS, vol. 108, 1988, page 171, résumé no. 32793a, Columbus, Ohio, US; C.A. BOUCHER et al.: "Pseudomonas solanacearum genes controlling both pathogenicity on tomato and hypersensitivity on tobacco are clustered", & J. BACTERIOL. 1987, 169(12), 5626-32 * Résumé * | 1-6 | |
| Y | IDEM ---- | 7,8 | **DOMAINES TECHNIQUES RECHERCHES (Int. Cl.4)** |
| Y | EP-A-0 172 914 (JAPAN TOBACCO) * En entier * ---- -/- | 7,8 | |

Le présent rapport a été établi pour toutes les revendications

| Lieu de la recherche | Date d'achèvement de la recherche | Examinateur |
|---|---|---|
| LA HAYE | 04-09-1989 | MADDOX A.D. |

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | CHEMICAL ABSTRACTS, vol. 106, 1987, page 159, résumé no. 150413h, Columbus, Ohio, US; J.J. SHAW et al.: "Direct analysis of the invasiveness of Xanthomonas campestris mutants generated by Tn4431. A transposon containing a promoterless luciferase cassette for monitoring gene expression", MOL. GENET. PLANT-MICROBE INTERACT., PROC. INT. SYMP., 3rd 1986 (Pub. 1987), 57-60 <br> * Résumé * <br> --- | 4 | |
| A | CHEMICAL ABSTRACTS, vol. 101, 1984, page 330, résumé no. 87079h, Columbus, Ohio, US; C.A. HENDRICK et al.: "Lipopolysaccharide-defective mutants of the wilt pathogen Pseudomonas solanacearum", & APPL. ENVIRON. MICROBIOL. 1984, 48(1), 94-101 <br> * Résumé * <br> --- | 1-6 | |
| A | BIOLOGICAL ABSTRACTS/RMM, résumé no. 47630; R.J. McLAUGHLIN et al.: "Potato seedpiece treatment with an avirulent variant of pseudomonas-solanacearum reduces incidence of bacterial wilt an brown rot", & PHYTOPATHOLOGY(US) 1985. vol. 75, no. 10, p1178 <br> * Résumé * <br> ---        -/- | 7,8 | DOMAINES TECHNIQUES RECHERCHES (Int. Cl.4) |

Le présent rapport a été établi pour toutes les revendications

| Lieu de la recherche | Date d'achèvement de la recherche | Examinateur |
|---|---|---|
| LA HAYE | 04-09-1989 | MADDOX A.D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.................................................................
& : membre de la même famille, document correspondant

EPO FORM 1503 03.82 (P0402)

Office européen
des brevets

**RAPPORT DE RECHERCHE EUROPEENNE**

Numero de la demande

EP 89 40 1422

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | BIOLOGICAL ABSTRACTS, vol. 85, 1988, colonne de droite, résumé no. 78511, Philadelphia, PA, US; XU et al.: "Molecular cloning of genes that specify virulence in Pseudomonas solanacearum", & J. BACTERIOL 170(2): 617-622. 1988 <br> * Résumé * <br> ----- | 1-6 | |

DOMAINES TECHNIQUES RECHERCHES (Int. Cl.4)

Le présent rapport a été établi pour toutes les revendications

| Lieu de la recherche | Date d'achèvement de la recherche | Examinateur |
|---|---|---|
| LA HAYE | 04-09-1989 | MADDOX A.D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
............................................................
& : membre de la même famille, document correspondant

EPO FORM 1503 03.82 (P0402)